# EUROPEAN PATENT SPECIFICATION

(11) **EP 0 436 545 B1**
(45) Date of publication and mention of the grant of the patent: **13.03.1996**
(21) Application number: 89906520.5
(22) Date of filing: 04.05.1989
(51) Int. Cl.: A61K 39/00, A01N 63/02, A01N 37/48, C12Q 1/70, C12P 21/00, C12N 9/00, G01N 33/53

(54) **THERAPEUTIC METHODS USING CATALYTIC ANTIBODIES**
HEILUNGSVERFAHREN UNTER VERWENDUNG KATALYTISCHER ANTIKÖRPER
PROCEDES THERAPEUTIQUES UTILISANT DES ANTICORPS CATALYTIQUES

(30) Priority: 04.05.1988 US 190271
(43) Date of publication of application: 17.07.1991
(60) Divisional application: 95111577.3
(73) Proprietor: IGEN, INC., Gaithersburg, Maryland 20877 (US)
(72) Inventor: POWELL, Michael, J., Gaithersburg, MD 20852 (US); REES, Anthony, R., Rockville, MD 20852 (US); MASSEY, Richard, J., Rockville, MD 20852 (US)
(74) Representative: Ritter, Stephen David
(86) International application number: US8901950
(87) International publication number: WO8910754

(56) References cited:
- EP-A- 0 251 093
- EP-A- 0 260 939
- US-A- 4 659 567
- US-A- 4 792 446
- Science, Vol. 234, pp. 1566-1570, Dec 1986; TRAMONTANO et al.: "Catalytic Antibodies." (See especially p. 1569.)
- Science, Vol. 234, pp. 1570-1573, Dec 1986; POLLACK et al.: "SelectiveChemical Catalysis by Antibody:" (See entire article.)
- Journal of the American Chemical Society, Vol. 95:5, pp. 1621-1628, March 1973; RASO et al.: "Antibodies specific for conformationally distinct coenzyme-substrate transition state analogs..."
- Journal of the American Chemical Society, Vol. 109, pp. 2174-6, April 1987; JACOBS et al.: "Catalytic Antibodies."
- Science, Vol. 237, pp. 1041-1043, Aug 1987; NAPPER et al.: "A Sterospecific Cyclization by an Antibody".
- Journal of Cellular Biochemistry, Vol. 11c (supplem), p. 238, March 1987; TRAMONTANO et al.: "Specificity and Mechanism of Esterolytic Antibodies."
- Scientific American 258, 42-50 (1988)

## Description

This invention relates to the manufacture of pharmaceutical compositions for administration resulting in in vivo activation of a prodrug.

Several publications are referenced in this application by Arabic numerals within parentheses. Full citation for these references are found at the end of the specification immediately preceding the claims. The references more fully describe the state of the art to which this invention pertains as well as certain aspects of the invention itself.

### BACKGROUND OF THE INVENTION

There are numerous enzymes which have been identified as capable of catalyzing various chemical reactions. Similarly, it has been discovered that antibodies can be elicited to catalyze a variety of chemical reactions (U.S. App. Ser. No. 674,253). It is well known that antibodies and enzymes share a fundamental similarity in that both are specialized proteins that bind to other molecules. However, there are important physiological differences between antibodies and enzymes.

Antibodies typically bind to a molecule or antigen so that the antigen is marked as foreign to the organism that produced the antibody. The binding of the antibody to the antigen enables the antigen to be removed from the organism. Enzymes are biological catalysts which bind a molecule in such a way that the activation energy of a reaction involving a molecule or substrate is lowered, thereby increasing the rate of the reaction.

Linus Pauling hypothesized that there are two types of interactions between proteins and the molecules that bind them. Antibodies bind molecules in their ground states most strongly while enzymes bind molecules in higher energy states most strongly.

Pauling attempted to explain the mechanism of enzyme catalysis based upon such binding. During the course of the chemical reaction, the reactants undergo one or more transitions through intermediate structures or transition states which are energically less favorable than either the reactant or the product. The hydrolysis reaction of a peptide linkage or an ester bond in an aqueous medium passes through a tetrahedral carbon transition state. In the transition state, a tetrahedral carbon atom is bonded to: a carbon atom of the acid portion of the peptide linkage or ester bond; two oxygen atoms, one corresponding to the carbonyl group and the other corresponding to a hydroxyl ion or water molecule of the medium; and either the oxygen atom of the alcohol portion of an ester or the nitrogen atom of the amine portion of the peptide linkage. The transition state can be neither isolated nor detected since it exists for only about 10⁻¹³ seconds.

In molecular terms, these transition states reflect changes in bond lengths and bond angles as well as the formation and breakage of bonds. The energy required to achieve a transition state is denoted as the activation energy which may also be considered as the difference in energy between the energy of the transition state and the energy of the reactants. According to Pauling's hypothesis, an enzyme preferentially binds the transition state of a reaction, thereby stabilizing it relative to the substrate and products and reducing the activation energy of the reaction, thus increasing the reaction rate.

By extending this explanation, Pauling also predicted that stable analogs of a transition state would bind tightly to an enzyme. In a discussion of substrate distortion as one of several possible sources of rate enhancement by enzymes, it has been suggested that the term "transition state analog" might be used to describe an inhibitor of this kind (1).

Pauling's prediction has become the basis for the now well established approach to enzyme inhibitor design. The strategy for designing enzyme inhibitors has suggested a strategy for preparing catalytic antibodies whereby antigens are designed based upon mechanistic principles so that antibodies raised in response to such antigens will catalyze a chemical reaction by carrying out the reaction mechanism implicit in the design of the antigen. This strategy has been attempted a number of times.

For example, a transition state analog mimicking the transition state for ester bond cleavage was used to elicit a monoclonal antibody which acted as a catalytic esterase (2). Specifically, the monoclonal antibody elicited accelerated by a factor of 15,000 the hydrolysis of an aryl ester of acetic acid.

In another example, a transition state analog mimicking an intramolecular 6-member ring cyclization transition state was used to elicit a monoclonal antibody which acted as a stereospecific, enzyme-like catalyst (3). Specifically, the monoclonal antibody so elicited accelerated, by about a factor of 800, the formation of a single enantiomer of a δ-lactone from the corresponding racemic δ-hydroxyester.

Similarly, monoaryl phosphonamidate esters, designated as analogs of the transition state in the hydrolysis of aryl amides, were synthesized and used as haptens to elicit specific monoclonal antibodies capable of catalyzing the hydrolysis of aryl amides (4). Certain of the antibodies elicited were reportedly found to be catalytic and selective for the hydrolysis of particular aryl esters, with a rate acceleration of 250,000.

Phosphonamidates or phosphonate analog-ligands having conformations that substantially correspond to the conformation of a hydrolytic transition state of an amide or ester ligand and which have been used to produce antibodies are described in U.S. Patent 4,659,567 to Tramontano et al. (Tramontano). Antibodies so produced purportedly include a paratope that binds to and stabilizes the tetrahedral carbon atom of the ester hydrolysis transition state of the ligand to hydrolyze the ligand at a predetermined site.

Analog-ligands which can be used to produce antibody catalysts for the hydrolysis of esters and amides are also described in European Patent Application 0,251,093 of Kollmorgen Corp (Kollmorgen).

Analog ligands have been designed in accordance with a rational design approach which maximizes stabilization of the transition state and optimizes atomic relationships within the proteolytic transition state analog, thereby enabling the elicitation of antibodies capable of producing the two dramatic effects of enzyme catalysis, i.e., molecular recognition and rate acceleration. Antigens, immunogens, and immunological processes for the production of catalytic antibodies are disclosed in commonly assigned parent application Ser. No. 190,271, filed May 4, 1988, the contents of which are incorporated by reference, and in commonly owned application Ser. No. , filed on even date with this application, the contents of which are likewise incorporated by reference.

It is known that antibodies raised against peptides are able to bond to the same sequence when the latter are located within an intact protein. For example, antibodies elicited against a peptide comprising amino acids 1-15 of tumor necrosis factor (TNF) are able to bind to native tumor necrosis factor and in doing so, inhibit its interaction with a cell surface receptor (1). Similarly, antibodies against a peptide comprising amino acids of the gp 120 coat protein from HIV cross-react with the intact virus and inhibit the interaction of the virus with its cellular receptor, CD4 (2). In another example, monoclonal antibodies raised against a peptide comprising amino acids 67-83 of hen egg lysozyme were able to cross-react with the intact protein and are able to recognize other avian species of lysozyme whose sequences within the epitope are substantially similar (3).

While methods for preparing catalytic antibodies have been described, and while methods for binding noncatalytic antibodies to antigens or substrates of interest have been described, the art has heretofore not provided therapeutic methods for directing rate-enhancing antibodies against selected target biomolecules. Moreover, the art has not provided methods for decreasing or otherwise controlling the biological effects of certain undesirable proteins, e.g., tumor necrosis factor, nor has the art provided methods for reducing the symptoms of allergies, reducing the infectivity of HIV virus, decreasing the effects of hypertension and other disease and physiological conditions which can be controlled by cleaving or forming certain biomolecules.

Scientific American, 258, pages 42-50 (1988) suggests using catalytic antibodies to cleave viral coat proteins.

EP-A-0260939 also proposes catalytic antibodies for cleavage of proteins, such as by cleavage of amide or ester bonds. Other applications of catalytic antibodies, namely carbohydrate degradation, treatment of disease and anticancer therapy, are mentioned generally in EP-A-0251093.

British Journal of Cancer, 56, pages 531-532 (1987) describes the desirability of targetting cancers with an enzyme that matches a prodrug. A later publication in British Journal of Cancer, 58, pages 700-703 (1988) describes generating a cytotoxic mustard at tumour sites using carboxypeptidase linked to an antibody.

The art has not however provided reliable methods for the activation of prodrugs using catalysts to release the active drug form from the prodrug.

The invention provides according to specific aspects thereof
1. The use of a catalytic monoclonal antibody in the manufacture of a pharmaceutical composition for in vivo activation of a prodrug by a procedure comprising:
   (a) introducing a prodrug into a patient, said prodrug having a chemical bond therein which upon cleavage releases the active form of said drug; and
   (b) introducing into said patient said catalytic monoclonal antibody being capable of catalytically cleaving said bond in said prodrug;
2. The use of a prodrug in the manufacture of a pharmaceutical composition for in vivo activation of a prodrug by a procedure comprising:
   (a) introducing the prodrug into a patient, said prodrug having a chemical bond therein which upon cleavage releases the active form of said drug; and
   (b) introducing into said patient a catalytic monoclonal antibody capable of catalytically cleaving said bond in said prodrug;
3. The use of a prodrug in the manufacture of a pharmaceutical composition for in vivo activation of a prodrug by a procedure comprising:
   (a) introducing the prodrug into a patient, said prodrug having a chemical bond therein which upon cleavage releases the active form of said drug; and
   (b) introducing into said patient an immunogen capable of eliciting within said patient an antibody capable of catalytically cleaving said bond in said prodrug; and
4. The use of an immunogen in the manufacture of a pharmaceutical composition for in vivo activation of the prodrug by a procedure comprising:
   (a) introducing a prodrug into a patient, said prodrug having a chemical bond therein which upon cleavage releases the active form of said drug; and
   (b) introducing into said patient the immunogen capable of eliciting within said patient an antibody capable of catalytically cleaving said bond in said prodrug.

The object of the invention is defined in the claims.

### DETAILED DESCRIPTION OF THE INVENTION

### Definition of Terms

In its broadest sense, the term "antigen" is defined as a molecule which induces the formation of an antibody. As used herein, the term "antigen" means a molecule which is inherently immunogenic, a hapten according to the invention or an immunogen which comprises a hapten according to the invention coupled to a carrier molecule by a suitable coupling moiety. Carrier molecules include, for example, keyhole limpet hemocyanin (KLH), thyroglobulin, chicken immunoglobulin, ovalbumin, bovine serum albumin (BSA), T-helper peptides, etc. "Coupling moieties" as used herein refer to biotechnological cross-linking reagents well known in the art (e.g., commercially available from Pierce, Rockford, Illinois) and include, for example, Trout's reagent, dissuccinyl suberate, etc.

The term "antibody" includes whole immunoglobulins and fragments thereof which contain the binding site for the antigen.

The term "transition state analog" as used herein refers to an array of atoms which is designed to approximate or "mimic" the configuration of an amide bond or an ester bond as such bonds exist in a hydrolytic transition state.

The term "dipeptide analog" as used herein refers to a structure which comprises a transition state analog or strained ground state analog or elements of both having side chains of two amino acids which are in positions analogous to those of the dipeptide being mimicked. In other words, in a dipeptide analog, the normal amide bond (i.e., -CO-NH-) between the two amino acids has been replaced by an array of atoms as defined above. Additional amino acid residues may be incorporated to surround the dipeptide analog to form a polypeptide. Thus, the dipeptide analog replaces the peptide bond "targeted" for cleavage in the substrate molecule. The moieties surrounding the dipeptide analog contain peptide bond linkages which can be altered such that the naturally occurring C=O group is replaced by NH, O, S, CH₂, CF₂ or C=S and/or the naturally occurring NH group is replaced by O, S, CH₂, CF₂, C=O or C=S. For example, the moieties can be retropeptides in which the C=O and NH groups of the amide bonds are interchanged.

The terms "some or all" refer to a portion of the target molecule including at least the amide, ester or glycosidic bond to be cleaved or all of the target molecule. For example, in an embodiment of the invention, haptens designed for the purpose of eliciting antibodies to catalyze the cleavage of a specific peptide bond in a protein molecule comprising a polypeptide of many amino acid residues, the dipeptide analog corresponding to the target peptide bond need only be surrounded by not more than about eight amino acid residues. However, if the target molecule is a relatively short peptide, it is advantageous to surround the peptide bond analog with all the amino acid residues of the target molecule. One of ordinary skill in the art will realize that the desired specificity, the nature of the target molecule and other factors will dictate the ideal number of amino acid residues needed to surround the dipeptide analog.

The term "substantially corresponds" refers to moieties which are similar in charge and/or size to moieties in the antigen containing the analog of the bond to be cleaved. Preferably, the moieties are identical in size and charge, although such identity is not necessary for the hapten of the invention. Haptens may contain one or more asymmetric centers and therefore exist in enantiomeric and/or diastereomeric forms. In general, haptens are obtained in the form of racemates or mixtures of diastereomers. If desired, techniques well known in the art for the separation of the mixtures into sterically homogeneous constituents may be used. Preparation of the optical isomers in a pure state is also possible by using sterically homogeneous starting materials.

The term "hapten" as used herein is defined as a molecule which can act as an epitope. Haptens incorporate an amide, peptide, ester, or glycosidic bond to be cleaved or formed.

Physiologically acceptable salts include salts of mineral acids, for example, hydrochloric acid, sulfuric acid, nitric acid and the like, salts of monobasic carboxylic acids such as, for example, acetic acid, propionic acid and the like, salts of dibasic carboxylic acids such as, for example, maleic acid, fumaric acid and the like, and salts of tribasic carboxylic acids such as, for example, citric acid and the like.

The term "naturally occurring amino acid" as used herein includes the twenty essential alpha-amino acids and other alpha-amino acids which may or may not be found in proteins. These amino acids include alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, 4-hydroxyproline, 5-hydroxylysine, epsilon-N-methyllysine, 3-methylhistidine, beta-alanine, gamma-aminobutyric acid, homocysteine, homoserine, citrulline, ornithine, canavanine, djenkolic acid and beta-cyanoalanine. An amino acid consists of a carbon atom to which is bonded an amino group, a carboxyl group, a hydrogen atom and a distinctive group referred to as a "side chain." The term "analog of said side chain" as used herein is defined as a side chain of a naturally occurring amino acid in which one or more moieties of the naturally occurring side chain is replaced by one or more different moieties which substantially corresponds to the naturally occurring moiety Those side chains containing a hydroxy group can be glycosylated, phosphorylated, sulphonylated or protected by a hydroxy protecting group. The hydroxy group of any of the side chains may be protected by any number of suitable hydroxy protecting groups well known in the art. These include, for example, a tertiary butyl group.

The term "terminal amino protecting group" means any group capable of protecting the terminal amino moiety of a peptide or amino acid. Therefore, terminal amino protecting groups include acetyl, succinyl, biphenylcarbonyl, benzoyl, t-butyloxycarbonyl, carbobenzyloxy, tosyl, dansyl, isovaleryl, phthalyl, 1-adamantanesulphonyl, acetimido, benzimido, amidino, carbamyl and the functional equivalents thereof.

The term "terminal carboxyl protecting group" means any group capable of protecting the terminal carboxyl moiety of a peptide or amino acid. Terminal carboxyl protecting groups include (C₁-C₉)alkyl, phenyl, substituted methyl esters such as methoxymethyl and phenacyl esters, 2- substituted ethyl esters such as cyclohexyl and allyl, substituted benzyl esters such as para-methoxybenzyl and para-bromobenzyl, amides such as piperidinyl and hydrazide and functional equivalents thereof.

The term "biomolecule" is defined as any molecule which affects a biological system in vivo or in vitro. Biomolecules may be synthesized by cells or chemically synthesized. Examples of biomolecules include proteins, glycoproteins, peptides, steroids, nucleic acids, and oligo- or polysaccharides. Also included are synthetic organic analogs of peptides, steroids, nucleic acids, etc. Pharmaceutically active compounds such as theophylline, capoten, cyclosporin, etc., are also considered to be biomolecules.

The term "accessible" means capable of combining with the combining site of an antibody.

A catalytic antibody is an antibody which is capable of changing the rate of a chemical reaction, all other conditions (e.g., temperature, reactant/substrate concentration, etc.) being the same, which does not enter into the chemical reaction and therefore is not consumed in the reaction, and which has the capability of converting multiple moles of reactant/substrate per mole of catalytic antibody. From a mechanistic viewpoint, it binds the reactant/substrate, effects the accelerated conversion of the reactant/substrate to the product and then releases the product, changes the rate of the chemical reaction without shifting the position of the equilibrium. The aforementioned definitions are characteristics of ideal catalysts. However, in practice, even the best of catalysts become poisoned or deactivated by contamination in the reaction system or as a result of chemical or physical destruction during the reaction process. For reasons well known in the art, the true operation of a catalyst may be obscured by components of the reaction system or by the condition of the reaction environment.

A stoichiometric antibody is an antibody which enhances the rate of the chemical reaction stoichiometrically, i.e., it enhances the rate of the reaction, but unlike a catalytic antibody, is stoichiometrically consumed during the reaction.

### Identification of Biomolecules and Sequences Therein as Targets for Therapeutic Action

In reported examples of catalytic monoclonal antibodies, the specificity of said antibodies is achieved because the contacted molecule, the substrate, is essentially identical to the immunogen with the exception of the transition state structure, which is present only in the immunogen. It has now been found that this approach can be extended to the design of an immunogen, or hapten, in order to elicit catalytic antibodies capable of catalyzing a reaction in the substrate when that substrate is part of a larger, more complex biological molecule.

For example, in the design of an oligopeptide hapten, the transition state analog is located somewhere within the sequence which leads to induction of a catalytic monoclonal antibody with a peptidase activity. The specificity of the antibody is determined by the amino acids flanking the transition state analog structure. The antibody so elicited will hydrolyze the peptide specifically, while peptides having similar but not identical sequences or structures would not be hydrolyzed. Such a catalytic antibody will also hydrolyze the peptide when that peptide is part of a protein, provided (a) the peptide sequence in question when present in a native protein is available for binding to the catalytic antibody, i.e., the epitope is surface located, and (b) if the peptide sequence in question is available, it is able to assume the conformation in the protein exhibited by the full oligopeptide in its free, substrate active form.

It has now been found that algorithms that identify peptide, ester, or glycosidic bonds within a biomolecule as "accessible" or "nonaccessible," or as "surface" or "nonsurface" can be used to assist the process of antigen design. Such information is available in various forms and with various levels of precision. First, if the three-dimensional structure of the biomolecule is known, then regions determined to be surface located by inspection of the structure can be identified as possible antigens Three-dimensional structures of all published protein structures are stored in the Brookhaven database (4) and are readily available, while three-dimensional structures of other biomolecules are stored in the Cambridge database.

If no structure is available, then similar information, though less precise, may be obtained by reference to a computer model or predicted structure of the biomolecule. This is particularly helpful when the biomolecule of interest is part of a family of homologous biomolecules, in which case, "knowledge-based" predictions (5) can be made, thus identifying surface located sequences.

At an even less precise level, algorithms can be employed that produce a hydrophilicity profile for the biomolecule. Regions of the biomolecule that contain a high proportion of hydrophilic or charged moieties will be likely candidates for a surface location.

Certain amino acid sequences are preferred targets when cleavage of the protein chain is the goal. For example, sequences containing the following amino acid combinations, ASN-GLY, ASN-PRO, ASP-GLY, and ASP-PRO, when present in a protein or peptide chain, are a preferred site of a catalytic cleavage. Similarly, sequences containing glutamic acid and glutamine, i.e., GLN-X and GLU-X wherein X is any amino acid, are preferred cleavage sites.

A further alternative for designing cleavage of a protein when no other information is available is to follow the purely random approach in which antibodies are raised to various peptides along the protein sequence until a region of cross-reactivity between antibody and protein is located.

In order to effect therapeutic applications of monoclonal antibody technology, the monoclonal antibody must bind to, and not dissociate from, its antigen. The antigen may be a soluble protein, virus, or other toxic molecule, or may be the surface of a cell. After binding, the antibody-antigen complex either triggers the activation of lytic enzyme cascades or is removed by phagocytic cells.

Catalytic antibodies, on the other hand, operate by a fundamentally different mechanism. Since a catalytic antibody, protease, for example, is able to "cut" the protein at its site of binding, the location and design of the epitope must be such that, on proteolysis and subsequent dissociation of the catalytic antibody from its target, the biological function of the target is irreversibly lost. Thus, a noncatalytic antibody directed against an epitope within the receptor binding region of gp 120 from HIV may inhibit binding of the virus to its CD4 receptor (4). This inhibition will arise because the large bulk of the antibody will prevent the virus and its receptor from coming into contact.

While a catalytic antibody protease directed against the same region of the virus may also lead to loss of receptor interaction, to insure loss of infectivity, a different set of events must ensue. The protease will not remain bound after cutting the virus protein chain and therefore the act of cutting itself must lead to loss of binding to be effective. This may not necessarily happen. For example, cleavage of the particular peptide bond may not lead to sufficient disruption of the three-dimensional structure to destroy receptor binding activity. In contrast, proteolysis of a peptide bond within an epitope distant from the receptor binding region may lead to loss of infectivity by virtue of its destabilizing effect on the protein structure. Therefore, since catalytic antibody proteases do not act via a simple steric blocking effect, a radically different design process for the target sequences must be established. Particular examples of how this process may be implemented are described below.

The methods can also be used to effect a cleavage that leads to the activation of some biological function. Such reactions include the cleavage of peptide bonds, but may also include ester bonds or glycosidic bonds or other types of bonds.

One example of the cleavage of a biomolecule which leads to the activation of a biological function is the treatment of insulin-dependent diabetes. Patients self-administer insulin by injection. The art has searched for a formulation of insulin whose release into the circulation mimics the pharmacokinetics of the release of natural pancreatic insulin. Insulin exists in the pancreas in a pro-form, proinsulin, whose activity is many orders of magnitude lower than insulin itself. An antibody protease specific for the peptide bond that leads to conversion of proinsulin to insulin can be designed so that its kinetic characteristics allow release of insulin in vivo after injection of proinsulin plus antibody protease. This is an example of prodrug activation where the drug in this instance is a natural protein hormone. Prodrugs may include any therapeutically active molecule which leads to the activation of a biological function. The pro-form may either take advantage of a natural modification of the drug or any suitable synthetic modification thereof. Suitable drug derivatives with low activity (therapeutically beneficial or toxic), which, on modification with a suitable catalytic antibody, are converted to an active form. A particular example of this process is given below.

### Preparation and Use of Rate-Enhancing Antibodies

Rate-enhancing antibodies, i.e., stoichiometric and catalytic rate enhancers, may be elicited through both in vitro and in vivo techniques. The term "elicited" as used herein means elicitation of catalytic antibodies by antigens according to the invention through both in vitro and in vivo techniques. However, the skilled artisan will readily appreciate that when in vitro elicitation is involved, the haptens according to the invention, by themselves, may be used to elicit the catalytic antibodies. When elicitation is achieved through in vivo techniques, it is understood that immunogens comprising haptens complexed to a suitable carrier molecule are used to elicit the catalytic antibodies. The antigen comprises a hapten designed to elicit the appropriate hypervariable binding region in an antibody molecule to express intrinsic binding energy for the transition- state of a chemical reaction, particularly a hydrolytic reaction. Arrangement of amino-acid side chains generated in the combining-site will be appropriate for performing chemical modification of an epitope of interest. Additional improvements in catalytic efficiency can be achieved by site-directed mutagenesis.

Broadly, the method comprises exposing cells capable of producing antibodies to the antigen and thereby generating antibody producing cells; hybridizing the antibody producing cells with myeloma cells and thereby producing a plurality of hybridoma cells each producing monoclonal antibodies; and screening the plurality of monoclonal antibodies to identify a monoclonal antibody which catalyzes the chemical reaction of interest. The monoclonal antibody so identified may then be replicated, again by either in vivo or in vitro techniques, to obtain a quantity sufficient to catalyze the chemical reaction of interest.

The detection of antibodies with the desired catalytic activity and specificity is achieved by screening the hybridomas once they have been elicited. For example, screening may be achieved by high performance liquid chromatography (HPLC) or spectrophotometric methods (ELISA). Catalytic monoclonal antibodies are elicited "in vivo" by modification of the technique disclosed by Koprowski et al. in U.S. Patent No. 4,196,265, issued April 1, 1980, which is hereby incorporated by reference. The details of that process are known in the art. A series of monoclonal antibodies directed to a specific molecule are prepared under suitable conditions. This involves first immunizing BALB/C mice with an appropriate antigen. The antigen comprises a hapten according to the invention bound to a peptide or other carrier molecule.

Antibody-producing lymphocytes are then removed from the spleens of the immunized mice and hybridized with myeloma cells such as SP2/0 cells to produce hybridoma cells. These hybridoma cells are then plated in the wells of microtiter plates. The series of monoclonal antibodies being produced by the hybridoma cells is screened under appropriate conditions to identify monoclonal antibodies which catalyze the desired reaction under appropriate conditions. Alternatively, the medium may be tested for antibodies that bind to the immunogen and the hybridomas producing these antibodies then expanded in tissue culture or grown in vivo. Screening may be conveniently accomplished by treating a standardized solution of the reactant with an aliquot of medium withdrawn from a microtiter well and measuring the presence of the desired product by conventional instrumental methods. This measurement may be readily conducted, for example by spectrophotometric methods or by gas-liquid or high pressure liquid chromatography. By comparison with standardized samples of the desired product or reactant, rates of reaction may be quantified. In this manner, wells containing hybridoma cells producing catalytic monoclonal antibodies are identified. The selected hybridoma cells are then cultured to yield colonies.

These colonies may be further propagated in vitro or in vivo systems. In the latter case, mice such as syngeneic BALB/C mice are inoculated intraperitoneally with the selected hybridoma cells and produce tumors, generally within two or three weeks. These tumors are accompanied by the production of ascites fluid which contains the desired monoclonal antibodies. The monoclonal antibodies are then separately recovered from the ascites fluid by conventional methods such as ultrafiltration, ultracentrifugation, dialysis and immunaffinity chromatography.

The separately recovered monoclonal antibodies are introduced to an animal in vivo under suitable conditions permitting the formation of a complex between the monoclonal antibody and the target molecule. In general, the concentration of the catalytic antibodies used is less than the equivalent concentration of the target molecule and may be in the picomolar range. The antibodies should function under normal physiologic conditions in vivo. The skilled artisan will appreciate that the conditions suitable for complex formation may vary depending on the particular molecule and antibody under consideration.

Antibodies produced by any of the processes described above may be produced via an immortalized cell line, in vitro. A suitable form of the antibody (for example, a "humanized" mouse monoclonal antibody) would then be administered as a therapeutic "drug."

During the course of a chemical reaction, the reactants undergo one or more transitions through structures which are energetically less favorable than either the reactant or product. In molecular terms, these transition states (or intermediate structures) reflect changes in bond lengths and bond angles as well as formations and breakages of bonds. The energy required to achieve a transition state is denoted as the activation energy, which may also be considered as the difference in energy between the energy of the transition state and the energy of the reactants.

Catalysts increase chemical reaction rates by lowering the activation energy of a reaction. Antibodies elicited to a hapten or antigen, which antigens are chosen because, inter alia, they resemble the presumed transition state structure (i.e., a transition state analog, a strained ground state structure or both), can catalyze reactions. The antibody thus produced should stabilize the energy of the transition state relative to reactants and products. This approach has been successfully demonstrated in the generation of several catalytic monoclonal antibodies.

Catalytic antibodies elicited with rationally designed haptens are "site specific" in that they are deliberately designed only to catalyze cleavage of bonds having certain structural conformations at specific sites in a biomolecule. Likewise, these catalytic antibodies are designed only to catalyze the formation of bonds from the termini of moieties having certain structural conformations at those termini. Therefore, rationally designed haptens according to the invention may be used to elicit a site specific catalytic antibody capable of cleaving bonds at specific sites in a biomolecule to produce two or more cleavage products. The same catalytic antibody can then catalyze the formation of bonds wherein those cleavage products having the right structural conformation are joined.

Thus, the haptens are designed to mimic the transition states for a variety of chemical reactions. Preferably, the reactions are the cleavage or formation of a peptide, ester, amide, or glycosidic bond. For example, a hapten as shown below, 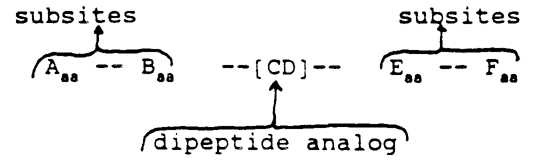
incorporates not only the dipeptide analog [CD] but also sub-site amino acid residues A, B, E, F. These subsite amino acid residues can be part of a cyclic structure as well as a linear structure. The optimum number of sub-site residues is determined by the size of the antibody combining site. It is likely that the only essential criterion for effective binding of antibody to a peptide is that complementarity between the antigen combining site of the antibody and the molecular surface of the binding peptide is maintained with regard to both shape and charge.

The haptens are designed in such a way such that antibodies raised against these haptens can selectively stabilize one or any of the high energy intermediates or transition states in the cleavage or formation of an amide, peptide, ester or glycosidic bond. These haptens fall into three general classes: one, those in which the hybridization of the atom corresponding to the carbonyl carbon of the scissile bond of the amide or ester bond is converted from sp² to sp³ hybridization; two, those in which any of the atoms corresponding to the amide, ester or glycosidic bond is replaced by a different atom; and three, those in which the atoms corresponding to the amide, ester or glycosidic bond are part of a monocyclic or bicyclic system.

Peptide sequences containing dipeptide analogs at the bond that is required to be hydrolyzed by the catalytic antibodies of one aspect of the present invention define a sequence that the catalytic antibody will hydrolyze in a native protein. The binding energy of the antibody is distributed in such a way as to allow both sequence specific recognition and chemical reactivity with the native protein or peptide of interest.

It has been reported that it is not necessary to prepare peptides longer than eight amino-acid residues (octapeptides) to demonstrate all continuous epitopes (5). It has also been demonstrated that antibodies bind to peptides in a reproducible manner (6). It has also been established that optical isomerism of the amino acids used has a powerful influence on the strength and specificity of antibody binding by dipeptides. Consequently, the importance of L and D amino acid residues in the immunizing antigen will have a profound effect on the chirality of the antibody combining site generated. In generating catalytic antibodies according to one aspect of the invention with predetermined specificity for particular sequential (continuous) or assembled epitopes in a native protein, the relationship between measurable properties of a protein and its immunogenic sites are important (7). With the ready availability of protein sequences, the most widely used algorithm is based on the likelihood of finding a sequential epitope at the site of a local maximum in the hydrophilicity profile (8). Surface accessibility profiles (9) and protein flexibility (10) also provide information on the antigen sites in a native protein sequence. With knowledge of these sites and the importance of these epitopes in receptor mediated interactions or other disease associated mechanisms, peptide haptens having dipeptide analogs within these important "bioactive" epitopes can be designed in accordance with the invention. The catalytic antibodies elicited with these haptens can then be utilized, for example, to digest epitopes on viral proteins or tumor derived growth factors or other peptides involved in life-threatening situations (e.g., tumor necrosis factor in bacterial sepsis, etc.).

Thus, the haptens are rationally designed from knowledge of mechanistic features of enzyme catalysis and provide suitable templates for generating antibody combining sites endowed with catalytic properties. Consequently, they incorporate all the necessary features to provide for antibodies capable of molecular recognition and catalytic function. Thus, a cyclic carbohydrate hapten of formula [1] would provide a good mimic of the proposed transition state [2] (11) for hydrolysis of a glycosidic bond in a typical O-linked glycoside [3], as illustrated below: 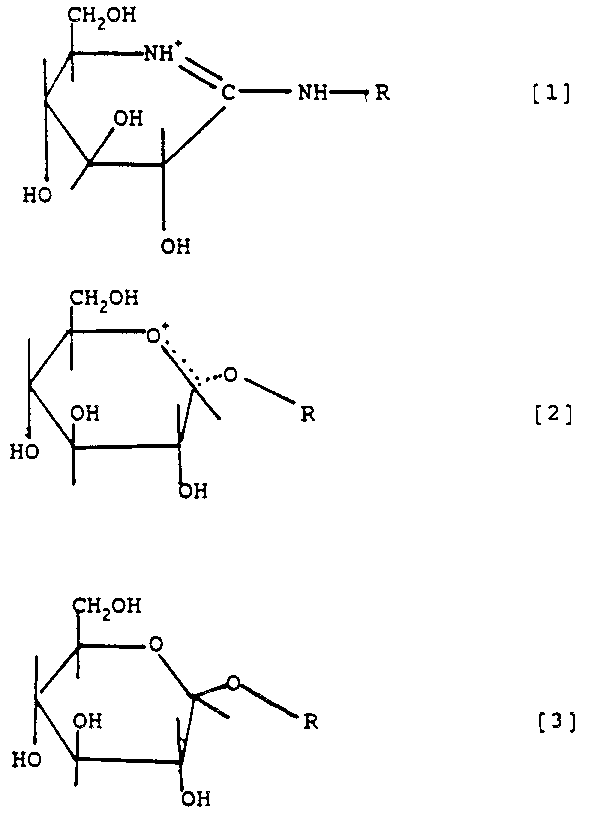
Catalytic antibodies have site-specific proteolysis capabilities in, e.g., the immunotherapy of viral infection. Viruses utilize their external coat proteins to attach to cellular receptors and invade the cell after attachment. For example, human immunodeficiency virus (HIV) uses a portion of the gp120 protein at its surface to attach to CD4 receptors on lymphocytes. The sequence for this cell attachment has been mapped to a region on the viral protein. With this information, antibodies can be generated by the methodology described in this invention to bind to this peptide sequence and cleave it in a site-specific manner. However, such antibodies preferably bind to the "native" sequence in the protein as opposed to a linear sequence (which would occur in a denatured protein). Thus, the antigenic determinants or epitopes in the "native" protein are often conformational (i.e., three-dimensional) rather than random linear arrangements. Here again, knowledge of epitopes on the protein is important in the design of antibodies having paratopes that can induce modifications of such epitopes. Therefore, haptens may be designed to have the same structural features of the epitopes, rather than random conformations. These structural features can be adopted by simple linear peptides, the lowest energy conformer being the preferred structure in solution. Secondary structural features may be introduced by crosslinking of amino-acid side chains or the use of -turn mimetics. Conformationally constrained haptens incorporating structures which are compatible with the epitope in the native protein may be essential for inducing the correct motif within the tertiary structure of the catalytic antibody hypervariable binding region. The advantages of conformationally constrained haptens are that they mimic the native structure in the protein and tend to mimic regions of the protein which are susceptible to cleavage.

### Preparation and Use of Antigens as Vaccines

It has also been found that advantage may be gained by exploiting the body's own ability to produce antibodies in vivo, in response to a certain antigenic stimulus. Processes are well known where the immune response to a particular pathogen can be primed by administration of either an inactive form of the pathogen or a peptide, antibodies against which cross-react with the pathogen. When the real pathogen is encountered, antibody-producing B-cells having the correct antibody specificity are already present. Such priming agents are commonly called vaccines (__). A similar process may be envisaged for induction of a catalytic antibody. In a process whereby the particular catalytic antibody to be induced is part of a systematic procedure, a hapten is designed to include a transition state analog for cleavage at a particular location. Immunization with this hapten would then activate certain B-cells, some of which would produce antibodies with catalytic activity. On exposure to the "normal" antigen (substrate), the primed cells in the animal would then respond giving rise to a catalytic antibody in vivo, thus obviating the need for parenteral administration. These "catalytic" vaccines might have very general applications in activation of catalytic antibodies having activities against viruses and other pathogens, toxic agents, drugs of abuse, naturally occurring proteins, therapeutically useful prodrugs.

The invention will be more fully described and understood with reference to the following illustrative examples.

### Example

### Activation of a Prodrug Using a Catalytic Antibody as a Glycosidase

### Background

Antimetabolites are compounds that interfere in either the biosynthesis, utilization, or metabolic function of normal cellular metabolites. To be successfully selective in the chemotherapy of tumors, an antimetabolite should adversely affect one or more vital metabolic reactions in the tumor without seriously endangering normal tissues.

Some of the most successful anticancer drugs have been those based on purine or pyrimidine analogs whose activity is dependent on their ability to inhibit DNA or RNA synthesis. One such drug is arabinosyl cytosine (I) (cytaribine, Ara C or CA) whose activity as an inhibitor of DNA synthesis derives from the presence of arabinose in place of ribose, the difference being in the stereochemistry of the 2' hydroxy group. Ara C is administered in the free 5'-hydroxl form and only becomes activated after entry into cells by phosphorylation to the 5'-triphosphate form. Thus, it is already a prodrug, but when administered systemically, its activation can take place in any cell, tumor or normal, into which the drug enters. As a result of the wide systemic distribution of the drug, numerous side effects occur, such as nausea, vomiting, alopecia, myelosuppression, etc.

It has now been found that Ara C can be modified to a prodrug form in which spontaneous intracellular activation would be reduced. First, an antitumor monoclonal antibody is targeted to the tumor, carrying with it a catalytic antibody, the two being either chemically or genetically linked. Second, the pro form of Ara C is administered and its activation is then restricted to those tissues bearing the abzyme activity. Thus a more favorable discrimination of tumor and normal tissue results. Since Ara C has a very short plasma half-life, diffusion of the activated drug away from the tumor site is followed by rapid deactivation before significant systemic toxicity results.

### Synthesis of 5'-galactosyl Ara C and its transition state analog

The synthesis of the amidine galactosyl analog of Ara C (15) is outlined in Schemes 2 and 3 below. The tribenzoylated derivative (3) from Scheme 1 is converted to (9) by treatment with methanesulfonyl chloride in pyridine followed by displacement with lithium azide in the N,NM-dimethylformamide at 75°C. Hydrogenation of (9) in ethanol at 50 psi of hydrogen pressure in the presence of 10% palladium on charcoal affords the 4'amino derivative.

The β-galactonolactam (13) is prepared by first treating 2,3,4,6-tetra-D-benzyl-2-d-galactopyranose (11) with dimethyl sulfoxide and acetic anhydride to give 2,3,4,6,-tetra-O-benzyl-D-Galactono-1,5-lactone (12) which is then condensed with aqueous ammonia (25% w/w) solution in the presence of trace amounts of Amberlite 1R 120 H⁺ in dioxane for 6 hrs to afford (13). Conversion of (13) to its imido ester analog by treating it with trimethyloxonium tetrafluorobarate followed by its reaction the 5'amino derivative (10) yields the fully protected amidine galactosyl analog (14). Deprotection using hydrogenation at 50 psi of hydrogen pressure in the presence of 10% palladium on charcoal followed by treatment with concentrated aqueous ammonia gives (15).

The synthesis of the 5'-beta-D-galactose analog of cytosine-beta-D-Arabinofuranoside (5) is outlined in Scheme 1.

Treatment of Ara C (1) with Bis (P-methoxyphenyl) phenyl methyl chloride in pyridine, followed by tribenzoylation using benzoyl chloride and then detritylation of (2) with trichloroacetic acid in dichloromethane, affords the partially protected derivative (3). Dilute aqueous acid treatment of beta-D-galactose pentaacetate (6) followed by treatment with sodium hydride and excess trichloroacetonitrile yields the trichloroacetimidate (8). Coupling of (8) with (3) in the presence of the Lewis acid boron trifluoride etherate in dichloromethase gives (4). The 5'-beta-D-galactose analog of Ara C (5) is obtained after the complete deprotection of (4) using concentrated aqueous ammonia.

### Production of antibodies and screening for Binding to Transition State Analogs

Monoclonal antibodies to (15) in Scheme 4, after conjugation to a suitable carrier, are produced essentially as described in Example 1. Antibody-producing clones are first screened for their ability to bind to the Ara C analog (15) by methods similar to those described in Example 1.

### In vitro catalytic activity assay

Those antibody clones displaying binding activity for (15) are screened for their ability to cleave the galactosyl moiety from the Ara C substrate (5) by an assay essentially as described in Koerner and Nieman (J. Chromatography 449, 216-228 (1988), but substituting galactose oxidase for glucose oxidase. The principle of the assay is the detection of galactose as it is released from the prodrug by the catalytic antibody using a galactose oxidase/luminol chemiluminescence procedure.

### In vivo assays

The conversion of galactosyl Ara C to Ara C by the catalytic antibody in the presence of target cells results in inhibition of DNA synthesis and cell-killing. A simple assay of DNA synthesis is carried out essentially as described in Gish et al. (J. Med. Chem 14, 1159-1162, 1971), in which the ablity of Ara C to inhibit DNA synthesis in phytohaemagglutin (PHA) stimulated human lymphocytes, using a tritiated thymidine incorporation assay, is measured.

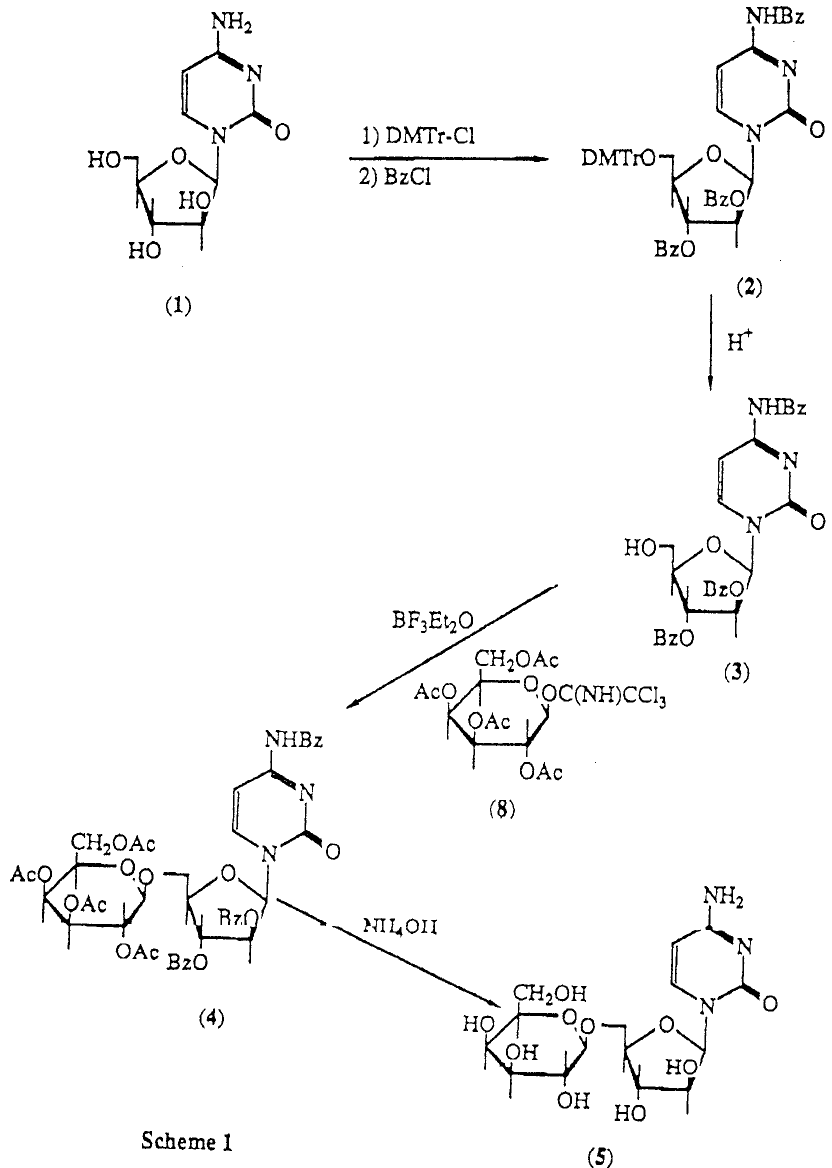

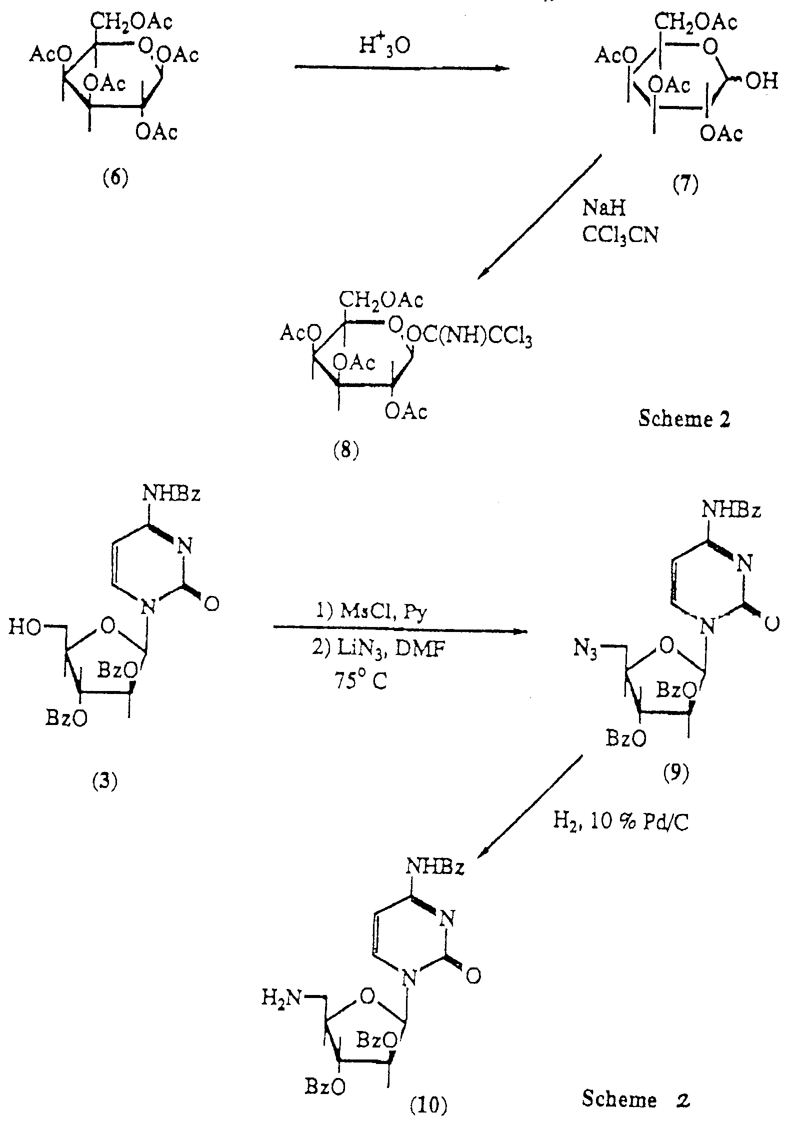

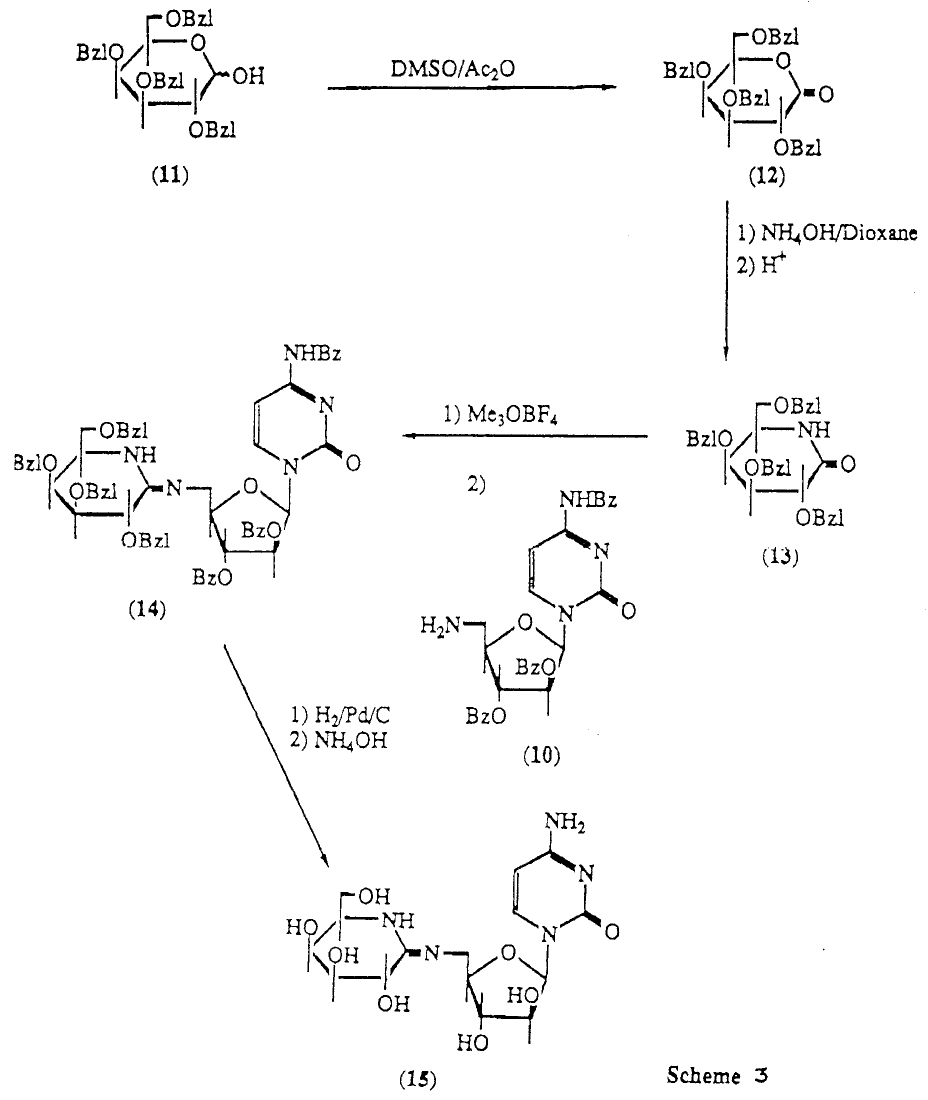

### REFERENCES

1. The Chemistry of Enzyme Action, Chapter 1, M.I. Page, Editor (Elsevier, Amsterdam 1984).
2. A.D. Napper et al., "A Stereospecific Cyclization Catalyzed by an Antibody," Science, 237, 1041-1043 (1987).
3. A. Tramontano et al., "Chemical Reactivity at an Antibody Binding Site Elicited By Mechanistic Design of a Synthetic Antigen," Proc. Nat'l Acad. Sci. USA, 83, 6736-6740 (1986).
4. H. White and W.P. Jencks, J. Biol. Chem., 252, p. 1688 (1976); H. White et al., ibid, 1700.
5. H.M. Geysen et al., J. Immunological Methods, 102, 259-274 (1987).
6. H.M. Geysen et al., Proc. Nat'l Acad. Sci. USA, 82, 178-182 (1985).
7. J.A. Berzofsky, Science, 229, 932-940 (1985).
9. T.P. Hopp and K.R. Woods, Proc. Nat'l. Acad. Sci, USA, 78, 3824-3828 (1981).
9. J. Novotny et al., Proc. Nat'l Acad. Sci., 226 (1986).
10. H.M. Geysen et al., Science, 235, 1184 (1878).
11. A. Fersht, Enzyme Structure and Mechanism, 2d ed., 433-436 (W.H. Freeman and Co., New York, 1985).
12. J. Blundell et al., Nature, 304, 273-275 (1983).
13. P. Corvol et al., J. Biol. Chem., 262(6), 2913-2918 (1987).
14. E. Atherton and R.J. Sheppard, J. Chem. Soc. Commun., 1151-1152 (1981).
15. M. Wilchek and S. Banniger, Methods Enzymol., 70, 151-159 (1980).
16. J. Menard and K.J. Catt, Endocrinology, 90, 422-430 (1972).
17. C.B. Pert et al., Proc. Nat'l. Acad. Sci, USA, 83, 9254-9258 (1986).
18. M.R. Pincus et al., Biochem. Biophys. Res. Commun., 143(a), 248-251 (1987).
19. D.M. Katz et al., Biochemistry, 18, 690-697 (1979).
20. Anderson et al., CPU, 10 27 (1977).
21. M.D. Schaff et al., Cell, 8, 405 (1976).
22. C. Milstein et al., Nature, 266, 550 (1977).
23. J.W. Littlefield, Expt'l. Cell Res., 41, 190 (1966).
24. D. Ho, J. Virol., 61, 2024 (1987).
25. B.D. Walker et al., Proc. Nat'l Acad. Sci. USA, 84, 8120 (1987).

## Claims

1. The use of a catalytic monoclonal antibody in the manufacture of a pharmaceutical composition for in vivo activation of a prodrug by a procedure comprising:
(a) introducing a prodrug into a patient, said prodrug having a chemical bond therein which upon cleavage releases the active form of said drug; and
(b) introducing into said patient said catalytic monoclonal antibody being capable of catalytically cleaving said bond in said prodrug.

2. The use of a prodrug in the manufacture of a pharmaceutical composition for in vivo activation of a prodrug by a procedure comprising:
(a) introducing the prodrug into a patient, said prodrug having a chemical bond therein which upon cleavage releases the active form of said drug; and
(b) introducing into said patient a catalytic monoclonal antibody capable of catalytically cleaving said bond in said prodrug.

3. The use according to Claim 1 or Claim 2 wherein said catalytic monoclonal antibody is linked to an antitumour monoclonal antibody.

4. The use according to Claim 3 wherein said pharmaceutical composition is for anticancer therapy.

5. The use according to Claim 3 wherein said use results in localized activation of said prodrug at targeted tissues.

6. The use according to Claim 5 wherein said targeted tissues are tumorous.

7. The use according to Claim 1 or 2 wherein said use results in systemic activation of said prodrug.

8. The use according to Claim 7 wherein said prodrug is proinsulin.

9. The use of a prodrug in the manufacture of a pharmaceutical composition for in vivo activation of a prodrug by a procedure comprising:
(a) introducing the prodrug into a patient, said prodrug having a chemical bond therein which upon cleavage releases the active form of said drug; and
(b) introducing into said patient an immunogen capable of eliciting within said patient an antibody capable of catalytically cleaving said bond in said prodrug.

10. The use of an immunogen in the manufacture of a pharmaceutical composition for in vivo activation of the prodrug by a procedure comprising:
(a) introducing a prodrug into a patient, said prodrug having a chemical bond therein which upon cleavage releases the active form of said drug; and
(b) introducing into said patient the immunogen capable of eliciting within said patient an antibody capable of catalytically cleaving said bond in said prodrug.

## Patentansprüche

1. Verwendung eines katalytischen monoklonalen Antikörpers bei der Herstellung einer pharmazeutischen Zubereitung zur *in vivo*-Aktivierung eines Prodrugs durch ein Verfahren, bei dem man
(a) das Prodrug in einen Patienten einbringt, wobei das Prodrug eine chemische Bindung aufweist, die bei ihrer Spaltung die aktive Form des Wirkstoffs freisetzt, und
(b) in den Patienten den katalytischen monoklonalen Antikörper einführt, der die besagte Bindung im Prodrug katalytisch spalten kann.

2. Verwendung eines Prodrugs bei der Herstellung einer pharmazeutischen Zubereitung zur *in vivo*-Aktivierung des Prodrugs durch ein Verfahren, bei dem man
(a) das Prodrug in einen Patienten einbringt, wobei das Prodrug eine chemische Bindung aufweist, die bei ihrer Spaltung die aktive Form des Wirkstoffs freisetzt, und
(b) in den Patienten den katalytischen monoklonalen Antikörper einführt, der die besagte Bindung im Prodrug katalytisch spalten kann.

3. Verwendung gemäß Anspruch 1 oder 2, bei der der katalytische monoklonale Antikörper mit einem monoklonalen Antikörper gegen Tumoren verknüpft ist.

4. Verwendung gemäß Anspruch 3, wobei die pharmazeutische Zubereitung für die Krebstherapie bestimmt ist.

5. Verwendung gemäß Anspruch 3, wobei die Verwendung zu einer lokalen Aktivierung des Prodrugs im Zielgewebe führt.

6. Verwendung gemäß Anspruch 5, wobei das Zielgewebe ein Tumorgewebe ist.

7. Verwendung gemäß Anspruch 1 oder 2, wobei die Verwendung zu einer systemischen Aktivierung des Prodrugs führt.

8. Verwendung gemäß Anspruch 7, bei der das Prodrug Proinsulin ist.

9. Verwendung eines Prodrugs bei der Herstellung einer pharmazeutischen Zubereitung zur *in vivo*-Aktivierung des Prodrugs durch ein Verfahren, bei dem man
(a) das Prodrug in einen Patienten einbringt, wobei das Prodrug eine chemische Bindung aufweist, die bei ihrer Spaltung die aktive Form des Wirkstoffs freisetzt, und
(b) in den Patienten ein Immunogen einführt, das im Patienten die Bildung von Antikörpern anregen kann, welche die besagte Bindung im Prodrug katalytisch spalten können.

10. Verwendung eines Immunogens zur der Herstellung einer pharmazeutischen Zubereitung zur *in vivo*-Aktivierung des Prodrugs durch ein Verfahren, bei dem man
(a) das Prodrug in einen Patienten einbringt, wobei das Prodrug eine chemische Bindung aufweist, die bei ihrer Spaltung die aktive Form des Wirkstoffs freisetzt, und
(b) in den Patienten das Immunogen einführt, das im Patienten die Bildung von Antikörpern induzieren kann, welche die besagte Bindung im Prodrug katalytisch spalten können.

## Revendications

1. Utilisation d'un anticorps monoclonal catalytique dans la fabrication d'une composition pharmaceutique pour l'activation in vivo d'une prodrogue par une procédure comprenant :
(a) l'introduction d'une prodrogue chez un patient, cette prodrogue comprenant une liaison chimique qui, par coupure, libère la forme active de ce médicament; et
(b) l'introduction chez ce patient de cet anticorps monoclonal catalytique qui est capable de couper catalytiquement cette liaison dans cette prodrogue.

2. Utilisation d'une prodrogue dans la fabrication d'une composition pharmaceutique pour l'activation in vivo d'une prodrogue par une procédure comprenant :
(a) l'introduction de la prodrogue chez un patient, cette prodrogue comprenant une liaison chimique qui, par coupure, libère la forme active de ce médicament; et
(b) l'introduction chez ce patient d'un anticorps monoclonal catalytique capable de couper catalytiquement cette liaison dans cette prodrogue.

3. Utilisation suivant les revendications 1 ou 2, dans laquelle cet anticorps monoclonal catalytique est lié à un anticorps monoclonal anti-tumeur.

4. Utilisation suivant la revendication 3, dans laquelle cette composition pharmaceutique est pour une thérapie anticancer.

5. Utilisation suivant la revendication 3, dans laquelle cette utilisation aboutit à une activation localisée de cette prodrogue au niveau des tissus ciblés.

6. Utilisation suivant la revendication 5, dans laquelle ces tissus ciblés sont tumoraux.

7. Utilisation suivant les revendications 1 ou 2, dans laquelle cette utilisation aboutit à une activation systémique de cette prodrogue.

8. Utilisation suivant la revendication 7, dans laquelle cette prodrogue est une proinsuline.

9. Utilisation d'une prodrogue dans la fabrication d'une composition pharmaceutique pour l'activation in vivo d'une prodrogue par une procédure comprenant :
(a) l'introduction de la prodrogue chez un patient, cette prodrogue comprenant une liaison chimique qui, par coupure, libère la forme active de ce médicament; et
(b) l'introduction chez ce patient d'un immunogène capable de générer chez ce patient un anticorps capable de couper catalytiquement cette liaison dans cette prodrogue.

10. Utilisation d'un immunogène dans la fabrication d'une composition pharmaceutique pour l'activation in vivo de la prodrogue par une procédure comprenant :
(a) l'introduction d'une prodrogue chez un patient, cette prodrogue comprenant une liaison chimique qui, par coupure, libère la forme active de ce médicament; et
(b) l'introduction chez ce patient de l'immunogène capable de générer chez ce patient un anticorps capable de couper catalytiquement cette liaison dans cette prodrogue.
